(19) Europäisches Patentamt
European Patent Office
Office européen des brevets

(11) EP 0 816 336 A1

(12) **EUROPÄISCHE PATENTANMELDUNG**

(43) Veröffentlichungstag:
**07.01.1998 Patentblatt 1998/02**

(51) Int. Cl.[6]: **C07D 207/26**, C07D 233/22, C11D 3/39

(21) Anmeldenummer: **97109809.0**

(22) Anmeldetag: **17.06.1997**

(84) Benannte Vertragsstaaten:
**AT BE CH DE DK ES FR IT LI NL**

(30) Priorität: **26.06.1996 DE 19625495**

(71) Anmelder: **Clariant GmbH**
**65929 Frankfurt am Main (DE)**

(72) Erfinder:
- **Löffler, Matthias, Dr.**
**65527 Niedernhausen (DE)**
- **Reinhardt, Gerd, Dr.**
**65779 Kelkheim (DE)**

# (54) Quartäre Ammoniumverbindungen als Bleichaktivatoren und deren Herstellung

(57) Verbindungen der allgemeinen Formel I

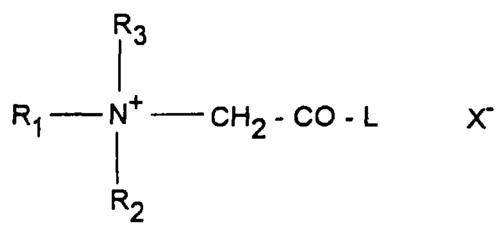

(I)

werden beansprucht, sowie ihre Herstellung und Verwendung, worin die Reste $R_1$, $R_2$ und $R_3$ organische Substituenten, L entweder ein Lactam oder ein cyclisches Amidin und X ein geeignetes Anion bedeuten. Die erfindungsgemäßen Verbindungen werden als Bleichaktivatoren in bleichenden Wasch- und Reinigungsmitteln eingesetzt.

EP 0 816 336 A1

Printed by Xerox (UK) Business Services
2.15.7/3.4

## Beschreibung

Diese Erfindung betrifft quartäre Ammoniumverbindungen, ihre Herstellung und Waschmittelzusammensetzungen, die diese quartären Ammoniumverbindungen als Bleichaktivatoren enthalten.

Es ist bekannt, daß das Bleichvermögen peroxidischer Bleichmittel, wie Perborate, Percarbonate, Persilicate und Perphosphate, verbessert werden kann, so daß die Bleichwirkung bei niedrigeren Temperaturen einsetzt, etwa bei oder unter 60°C, indem man die Vorstufen von bleichenden Peroxysäuren zusetzt, die oft als Bleichaktivatoren bezeichnet werden.

Viele Substanzen sind nach dem Stand der Technik als Bleichaktivatoren bekannt. Üblicherweise handelt es sich dabei um reaktive organische Verbindungen mit einer O-Acyl- oder N-Acyl-Gruppe, die in alkalischer Lösung zusammen mit einer Quelle für Wasserstoffperoxid die entsprechenden Peroxysäuren bilden.

Repräsentative Beispiele für Bleichaktivatoren sind etwa N,N,N',N'-Tetraacetylethylendiamin (TAED), Glucosepentaacetat (GPA), Xylosetetraacetat (TAX), Natrium-4-benzoyloxybenzolsulfonat (SBOBS), Natriumtrimethylhexanoyloxybenzolsulfonat (STHOBS), Tetraacetylglucoluril (TAGU), Tetraacetylcyansäure (TACA), Di-N-acetyldimethylglyoxin (ADMG) und 1-Phenyl-3-acetylhydantoin (PAH). Es sei beispielsweise auf GB-A-836 988, GB-A-907 356, EP-A-0 098 129 und EP-A-0 120 591 verwiesen.

Mittlerweile haben kationische Peroxysäure-Vorläufer, die neben O-Acyl- oder N-Acyl-Gruppen beispielsweise eine quartäre Ammoniumgruppe enthalten, an Bedeutung gewonnen, da sie hocheffektive Bleichaktivatoren sind. Solche kationischen Peroxysäure-Vorläufer sind beispielsweise in US-5 460 747, US-5 047 577, US-4 933 103, US-4 751 015, US-4 397 757, GB-1 382 594, WO-95 21150, EP-A-403 152, EP-A-427 224, EP-A-402 971, EP-371 809 und EP-A-284 292 beschrieben. Korrespondierende freie, stabile quartäre Persäuren sind z.B. in EP-A-340 754 und WO-94 01399 beschrieben.

Überraschenderweise wurde nun gefunden, daß bestimmte quartäre Bleichaktivatoren wie im folgenden beschrieben eine bessere Bleichwirkung aufweisen als die Bleichaktivatoren gemäß dem Stand der Technik. Zudem sind die erfindungsgemäßen Bleichaktivatoren mit weniger Syntheseschritten und somit ökologisch und ökonomisch günstiger herzustellen als die Bleichaktivatoren gemäß dem Stand der Technik.

Gegenstand der Erfindung sind Verbindungen der Formel I

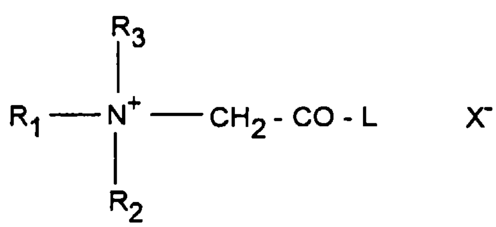

(I)

worin

a) $R_1$, $R_2$ und $R_3$ unabhängig voneinander $C_1$- bis $C_{24}$-Alkyl, Aryl, $C_2$-$C_{24}$-Alkenyl, $C_1$-$C_4$-Alkoxy-$C_1$-$C_4$-Alkyl, oder $CH_2$-CO-L sind,

oder

b) $R_2$ und $R_3$ zusammen mit dem Stickstoffatom, an das sie gebunden sind, einen Ring mit 4 bis 6 Kohlenstoffatomen bilden, wobei dieser Ring zusätzlich zum Stickstoffatom anstelle von Kohlenstoffatomen ein oder zwei Sauerstoffatome oder eine Gruppe

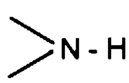 oder 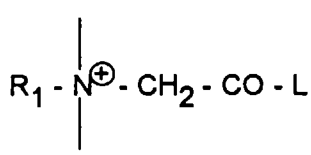

enthalten kann,

L eine Gruppe der Formeln

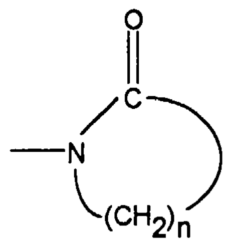 , oder 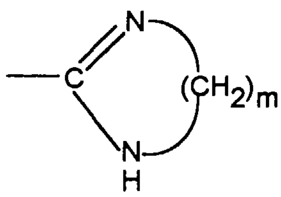

n eine Zahl von 3 bis 5,
m eine Zahl von 2 bis 4
und X ein Anion ist.

Bevorzugt als Anionen sind Chlorid, Bromid, Iodid, Fluorid, Sulfat, Hydrogensulfat, Carbonat, Hydrogencarbonat, Phosphat, Mono- und Di-Hydrogenphosphat, Pyrophosphat, Metaphosphat, Nitrat, Methosulfat, Dodecylsulfat, Dodecylbenzolsulfonat, Phosphonat, Methylphosphonat, Methandisulfonat, Methylsulfonat, Ethansulfonat und p-Toluolsulfonat.

Bevorzugt werden für die Substituenten $R_1$, $R_2$ und $R_3$ unabhängig voneinander unsubstituiertes $C_1$-$C_4$-Alkyl, unsubstituiertes $C_2$-$C_4$-Alkenyl oder Phenyl verwendet. L ist vorzugsweise eine Pyrrolidongruppe.

Besonders bevorzugt sind kurzkettige (Trialkylammonium)-acetylpyrrolidonchloride und entsprechende (Trialkylammonium)-acetylimidazolchloride mit $C_1$- bis $C_4$-Alkylgruppen, besonders 2-(N,N,N-Trimethylammonium)-acetylpyrrolidonchlorid, 2-(N,N,N-Triethylammonium)-acetylpyrrolidonchlorid, 2-(N,N-Diethyl-N-methylammonium)-acetylpyrrolidonchlorid sowie die entsprechenden Imidazol-Derivate.

Ein weiterer Gegenstand der Erfindung ist ein Verfahren zur Herstellung dieser Verbindungen. An Hand der folgenden allgemeinen Beispiele sollen die Synthesewege zu den erfindungsgemäßen kationischen Bleichaktivatoren dargestellt werden.

Allgemeines Beispiel 1:

Ein Lactam sowie eine geeignete Hilfsbase werden in einem organischem Lösemittel vorgelegt. Unter Kühlung wird bei Temperaturen zwischen 0 und 30°C, vorzugsweise bei 5°C, Chloracetylchlorid zugetropft. Nach einer Reaktionszeit von 3 bis 12 Stunden bei Temperaturen zwischen 10 und 30°C, vorzugsweise bei 25°C, wird der entstandene Niederschlag abfiltriert. Die weitere Umsetzung kann ohne vorherige Isolierung des entstandenen N-(Chloracetyl)amids erfolgen. Nach Zugabe eines tertiären Amins wird bei Temperaturen zwischen 20 und 100°C, vorzugsweise zwischen 70 bis 80°C zum entsprechenden 2-(N,N,N-Trialkylammonium)-Acetyllactamchlorid umgesetzt. Bei den vorzugsweise eingesetzten kurzkettigen Aminen wird gegebenenfalls in einem Autoklaven gearbeitet.

Allgemeines Beispiel 2:

Ein Lactam sowie eine geeignete Hilfsbase werden in einem organischem Lösemittel vorgelegt. Unter Kühlung wird bei Temperaturen zwischen 0 und 30°C, vorzugsweise bei 5°C, Chloracetylchlorid zugetropft. Nach einer Reaktionszeit von 3 bis 12 Stunden bei Temperaturen zwischen 10 und 30°C, vorzugsweise bei 25°C, wird der entstandene Niederschlag abfiltriert. Die weitere Umsetzung kann ohne vorherige Isolierung des entstandenen N-(Chloracetyl)amids erfolgen. Bei Temperaturen zwischen 0 und 50°C, vorzugsweise unter Kühlung bei 20°C, erfolgt die Zugabe eines sekundären Amins. Nach einer Reaktionszeit von 3 bis 12 Stunden bei Temperaturen zwischen 10 und 50°C, vorzugsweise bei 25°C wird der entstandene Niederschlag abfiltriert. Ohne daß eine vorherige Isolierung des entstandenen Zwischenproduktes erfolgen muß wird das erhaltene 2-(N,N-Dialkylamin)-Acetyllactam mit einem Alkylierungsmittel wie Methylchlorid oder Dimethylsulfat bei Temperaturen zwischen 20 und 100°C, vorzugsweise zwischen 70 und 80°C, zum entsprechenden 2-(N,N,N-Trialkylammonium)-Acetyllactam-Salz umgesetzt.

Bevorzugt ist es, als Hilfsbase die für die Reaktion eingesetzten Amine zu verwenden.

Gegenstand der Erfindung sind auch bleichende Wasch- und Reinigungsmittel, die die neuartigen Verbindungen als Bleichaktivatoren enthalten. Diese Wasch- und Reinigungsmittel enthalten neben einer Peroxyverbindung und dem kationischen Bleichaktivator üblicherweise auch oberflächenaktive Verbindungen und weitere Inhaltsstoffe.

Geeignete Peroxyverbindungen sind Alkaliperoxide, organische Peroxide wie Harnstoffperoxid, und anorganische Persalze, wie die Alkaliperborate, -percarbonate, -perphosphate, -persilicate und -persulfate. Mischungen aus zwei oder mehreren dieser Verbindungen sind ebenfalls geeignet. Besonders bevorzugt sind Natriumperborat-Tetrahydrat und insbesondere Natriumperborat-Monohydrat. Natriumperborat-Monohydrat ist wegen seiner guten Lagerbeständigkeit und seiner guten Löslichkeit in Wasser bevorzugt. Natriumpercarbonat kann aus Umweltschutzgründen bevorzugt sein.

Alkylhydroperoxide sind eine weitere geeignete Gruppe von Peroxyverbindungen. Beispiele für diese Stoffe sind Cumolhydroperoxid und t-Butylhydroperoxid.

In derartigen Wasch- und Reinigungsmitteln kann der erfindungsgemäße kationische Bleichaktivator mit einem Gewichtsanteil von etwa 0,1 % bis 20 %, bevorzugt von 0,5 % bis 10 %, insbesondere von 1 % bis 7,5 % vorhanden sein, zusammen mit einer Peroxyverbindung. Der Gewichtsanteil dieser Peroxyverbindungen beträgt gewöhnlich von 2 % bis 40 %, bevorzugt von 4 % bis 30 %, insbesondere von 10 % bis 25 %.

In den Wasch- und Reinigungsmitteln können neben den erfindungsgemäßen kationischen Bleichaktivatoren noch andere geeignete Bleichaktivatoren, wie beispielsweise TAED enthalten sein.

Die oberflächenaktive Substanz kann von Naturprodukten abgeleitet sein, wie etwa Seife, oder kann eine synthetische Verbindung aus der Gruppe der anionischen, nichtionischen, amphoteren, zwitterionischen oder kationischen oberflächenaktiven Substanzen sein, oder Mischungen aus diesen. Viele geeignete Substanzen sind kommerziell erhältlich, und sind in der Literatur beschrieben, beispielsweise in "Surface active agents and detergents", Vol. 1 und 2, von Schwartz, Perry und Berch. Der Gesamtanteil der oberflächenaktiven Verbindungen kann bis zu 50 Gew.-% betragen, vorzugsweise 1 Gew.-% bis 40 Gew.-%, insbesondere 4 Gew.-% bis 25 Gew.-%.

Synthetische anionische oberflächenaktive Substanzen sind üblicherweise wasserlösliche Alkalimetallsalze organischer Sulfate und Sulfonate mit Alkylresten von etwa 8 bis 22 Kohlenstoffatomen, wobei der Ausdruck "Alkyl" die Alkylsubstituenten höherer Arylreste einschließt.

Beispiele geeigneter anionischer Detergentien sind Natrium- und Ammoniumalkylsulfate, speziell die durch Sulfatierung höherer ($C_8$ bis $C_{18}$) Alkohole erhaltenen Sulfate; Natrium- und Ammoniumalkylbenzolsulfonate mit einem Alkylrest von $C_9$ bis $C_{20}$, insbesondere lineare sekundäre Natriumalkylbenzolsulfonate mit einem Alkylrest von $C_{10}$ bis $C_{15}$; Natriumalkylglycerinethersulfate, besonders die Ester der höheren, von Talg- und Kokosnußöl abgeleiteten Alkohole; die Natriumsulfate und -sulfonate der Kokosfettsäuremonoglyceride; Natrium- und Ammoniumsalze der Schwefelsäureester höherer ($C_9$ bis $C_{18}$) oxalkylierter, insbesondere der mit Ethylenoxid oxalkylierten Fettalkohole; die Reaktionsprodukte der Veresterung von Fettsäuren mit Isethionsäure und nachfolgender Neutralisierung mit Natriumhydroxid; Natrium- und Ammoniumsalze der Fettsäureamide des Methyltaurins; Alkan-Monosulfonate wie diejenigen aus der Reaktion von $\alpha$-Olefinen ($C_8$-$C_{20}$) mit Natriumbisulfit und diejenigen aus der Reaktion von Paraffinen mit $SO_2$ und $Cl_2$ mit anschließender basischer Hydrolyse, wobei ein Gemisch verschiedener Sulfonate entsteht; Natrium- und Ammoniumdialkylsulfosuccinate mit Alkylresten von $C_7$ bis $C_{12}$; und Olefinsulfonate, die bei der Reaktion von Olefinen, insbesondere $C_{10}$- bis $C_{20}$-$\alpha$-Olefinen, mit $SO_3$ und nachfolgender Hydrolyse der Reaktionsprodukte entstehen. Die bevorzugten anionischen Detergentien sind Natriumalkylbenzolsulfonate mit Alkylresten von $C_{15}$ bis $C_{18}$, und Natriumalkylethersulfate mit Alkylresten von $C_{16}$ bis $C_{18}$.

Beispiele für geeignete nichtionische oberflächenaktive Verbindungen, die bevorzugt zusammen mit anionischen oberflächenaktiven Verbindungen benutzt werden, sind insbesondere die Reaktionsprodukte von Alkylenoxiden (gewöhnlich Ethylenoxid) mit Alkylphenolen (Alkylreste von $C_5$ bis $C_{22}$), wobei die Reaktionsprodukte im allgemeinen 5 bis 25 Ethylenoxid-Einheiten (EO) im Molekül enthalten; die Reaktionsprodukte aliphatischer ($C_8$ bis $C_{18}$) primärer oder sekundärer, linearer oder verzweigter Alkohole mit Ethylenoxid mit im allgemeinen 6 bis 30 EO, und die Additionsprodukte von Ethylenoxid an Reaktionsprodukte aus Propylenoxid und Ethylendiamin. Andere nichtionische oberflächenaktive Verbindungen sind Alkylpolyglycoside, langkettige tertiäre Aminoxide, langkettige tertiäre Phosphinoxide und Dialkylsulfoxide.

Amphotere oder zwitterionische oberflächenaktive Verbindungen können ebenfalls in den erfindungsgemäßen Zusammensetzungen verwendet werden, was aber wegen deren hoher Kosten meistens nicht bevorzugt ist. Wenn amphotere oder zwitterionische Verbindungen verwendet werden, so geschieht das in der Regel in kleinen Mengen in Zusammensetzungen, die hauptsächlich anionische und nichtionische Tenside enthalten.

Auch Seifen können in den erfindungsgemäßen Zusammensetzungen verwendet werden, vorzugsweise mit einem Anteil von weniger als 25 Gew.-%. Sie sind besonders geeignet in geringen Mengen in binären (Seife/anionisches Tensid) oder in ternären Mischungen zusammen mit nichtionischen oder gemischten synthetischen anionischen und nichtionischen Tensiden. Die verwendeten Seifen sind bevorzugt die Natriumsalze, und weniger bevorzugt die Kaliumsalze gesättigter oder ungesättigter $C_{10}$- bis $C_{24}$-Fettsäuren, oder deren Mischungen. Die Anteile solcher Seifen können von 0,5 Gew.-% bis 25 Gew.-% betragen, geringere Mengen von 0,5 Gew.-% bis 5 Gew.-% sind im allgemeinen ausreichend zur Schaumkontrolle. Seifenanteile zwischen etwa 2 % und etwa 20 %, besonders zwischen etwa 5 % und etwa 10 %, haben einen positiven Effekt. Dieses ist besonders der Fall in hartem Wasser, wo die Seife als Zusätzliche Buildersubstanz dient.

Die Wasch- und Reinigungsmittel enthalten im allgemeinen auch einen Builder.

Als Builder kommen in Betracht: Calcium bindende Stoffe, Fällungsmittel, Calciumspezifische Ionentauscher und deren Mischungen. Beispiele für Calcium bindende Stoffe umfassen Alkalimetallpolyphosphate, wie Natriumtripolyphosphat; Nitrilotriessigsäure und ihre wasserlöslichen Salze; die Alkalimetallsalze der Carboxymethyloxybernsteinsäure, Ethylendiamintetraessigsäure, Oxydibernsteinsäure, Mellithsäure, Benzolpolycarbonsäuren, Zitronensäure; und Polyacetalcarboxylate, wie in US-4 144 226 und US-4 146 495 offenbart.

Beispiele für Fällungsmittel sind Natriumorthophosphat, Natriumcarbonat und Seifen aus langkettigen Fettsäuren.

Beispiele für Ionentauscher, die für Calcium spezifisch sind, sind die verschiedenen Arten wasserunlöslicher, kristalliner oder amorpher Aluminiumsilicate, von denen die Zeolithe die bekanntesten Vertreter sind.

Diese Buildersubstanzen können von 5 Gew.-% bis 80 Gew.-% vorhanden sein, bevorzugt ist ein Anteil von 10 Gew.-% bis 60 Gew.-%.

Neben den bereits erwähnten Inhaltsstoffen können die Wasch- und Reinigungsmittel jeden der konventionellen Zusatzstoffe in Mengen enthalten, die man üblicherweise in solchen Mitteln vorfindet. Beispiele dieser Zusatzstoffe umfassen Schaumbildner, wie etwa Alkanolamide, besonders die Monoethanolamide aus Palmkernöl-Fettsäuren und Kokosnuß-Fettsäuren; schaumverhindernde Substanzen, wie etwa Alkylphosphate und -silicone; Vergrauungsinhibitoren und ähnliche Hilfsmittel, wie etwa Natriumcarboxymethylcellulose und Alkyl- oder substituierte Alkylcelluloseether; Stabilisatoren, wie Ethylendiamintetraessigsäure; Weichmacher für Textilien; anorganische Salze, wie Natriumsulfat; und, in üblicherweise kleinen Mengen, fluoreszierende Stoffe, Parfüme, Enzyme wie Proteasen, Cellulasen, Lipasen und Amylasen, Desinfektionsmittel und Farbstoffe. Die Bleichaktivatoren dieser Erfindung können in einer Vielzahl von Produkten eingesetzt werden. Diese umfassen Textilwaschmittel, Textilbleichmittel, Oberflächenreiniger, Toilettenreiniger, Geschirrspülmaschinenreiniger, und auch Gebißreiniger. Die Waschmittel können in fester Form oder flüssiger Form vorliegen.

Es ist aus Gründen der Stabilität und Handhabbarkeit vorteilhaft, die Bleichaktivatoren in Form von Granulaten zu verwenden, die neben dem Bleichaktivator ein Bindemittel enthalten. Verschiedene Methoden, solche Granulate herzustellen, sind in der Patentliteratur beschrieben, so beispielsweise in CA-1 102 966, GB-1 561 333, US-4 087 369, EP-A-0 240 057, EP-A-0 241 962, EP-A-0 101 634 und EP-A-0 062 523. Jede dieser Methoden ist für die erfindungsgemäßen Bleichaktivatoren anwendbar.

Die die Bleichaktivatoren enthaltenden Granulate werden im allgemeinen der Waschmittelzusammensetzung zusammen mit den anderen, trockenen Bestandteilen wie etwa Enzymen, anorganischen Peroxidbleichmitteln zugesetzt. Die Waschmittelzusammensetzung, zu der die Aktivatorgranulate zugegeben werden, kann auf verschiedene Wegen erhalten werden, wie etwa durch Trockenmischen, Extrudieren oder Sprühtrocknung.

In einer weiten Ausführungsform sind die erfindungsgemäßen Bleichaktivatoren besonders geeignet für nicht wäßrige, flüssige Waschmittel, zusammen mit einer bleichenden Peroxyverbindung, etwa Natriumperborat, um dem Waschmittel ein großes Reinigungsvermögen für Gewebe und Textilien zu verleihen. Derartige nicht wäßrige, flüssige Waschmittel, die pastöse und gelatinöse Detergentienzusammensetzungen mit einschließen, sind im Stand der Technik bekannt, und sind beispielsweise in US-2 864 770, US-2 940 938, US-4 772 412, US-3 368 977, GB-A-1205 711 GB-A-1 370 377, GB-A-1 270 040, GB-A-1 292 352, GB-A-2 194 536, DE-A-2 233 771 und EP-A-0 028 849 beschrieben.

Es handelt sich dabei um Zusammensetzungen in Form eines nichtwäßrigen, flüssigen Mediums, in dem eine feste Phase dispergiert sein kann. Das nicht wäßrige, flüssige Medium kann eine flüssige, oberflächenaktive Substanz sein, vorzugsweise eine nichtionische oberflächenaktive Substanz; ein nicht polares flüssiges Medium wie etwa flüssiges Paraffin; ein polares Lösungsmittel, wie etwa Polyole, zum Beispiel Glycerin, Sorbitol, Ethylenglycol, eventuell in Verbindung mit niedermolekularen einwertigen Alkoholen wie Ethanol oder Isopropanol; oder Mischungen daraus.

Die feste Phase kann aus Buildersubstanzen, Alkalien, abrasiven Stoffen, Polymeren, anderen festen ionischen oberflächenaktiven Stoffen, Bleichmitteln fluoreszierenden Stoffen und anderen üblichen festen Inhaltsstoffen bestehen.

Die folgenden Beispiele sollen einen Überblick über die Ausführungsformen der Erfindung geben.

Beispiel 1:

a) Synthese von N-(Chloracetyl)-pyrrolidon

Zu 81,0 g 2-Pyrrolidon, vorgelegt in 200 ml Toluol, werden 54,7 g Chloracetylchlorid unter Eiskühlung zugetropft, so daß die Temperatur des Reaktionsgemisches bei 5°C bleibt. Nach erfolgter Zugabe wird das Reaktionsgemisch 6 Stunden bei Raumtemperatur gerührt. Der entstandene Niederschlag wird abgesaugt und das Lösungsmittel wird im Vakuum entfernt. Es resultieren 74,5 g N-(Chloracetyl)-pyrrolidon, entsprechend 97 % Ausbeute.

b) Synthese von 2-(N,N-Diethylamin)-Acetylpyrrolidon

Zu 80,0 g (Chloracetyl)-pyrrolidon, gelöst in 200 ml Toluol, werden 73,1 g Diethylamin unter Eiskühlung zugetropft, so daß die Temperatur des Reaktionsgemisches 50°C nicht übersteigt. Nach erfolgter Zugabe wird das Reaktionsgemisch auf Raumtemperatur abgekühlt und bei dieser Temperatur weitere 2 Stunden gerührt. Der entstandene Niederschlag wird abgesaugt und das Lösungsmittel wird im Vakuum entfernt. Es resultieren 74,9 g 2-(N,N-Diethylamin)-Acetylpyrrolidon, entsprechend 76 % Ausbeute.

c) Synthese von 2-(N,N-Diethyl,N-Methylammonium)-Acetylpyrrolidontosylat (Verbindung Nr. 1)

Zu 19,8 g 2-(N,N-Diethylamin)-Acetylpyrrolidon in 30 ml Acetonitril werden 73,1 g Toluol-4-sulfonsäuremethylester zugetropft. Nach erfolgter Zugabe wird 3 Stunden auf Rückfluß erhitzt, anschließend wird das Lösungsmittel im Vakuum entfernt. Es resultiert ein hochviskoses Öl, welches nach Umkristallisation aus Isopropanol in Form von farblosen Kristallen anfällt. Ausbeute an 2-(N,N-Diethyl,N-Methylammonium)-Acetylpyrrolidontosylat:

33,5 g entsprechend 87 % Ausbeute.

In analoger Weise wurden die Verbindungen 2 und 3 hergestellt (siehe Seite 13).

Beispiel 2:

Durch Zusammengeben von 200 ml einer wäßrigen Lösung von 5 g/l Referenzwaschmittel (WMP), erhalten von WFK-Testgewebe GmbH, Krefeld, 150 mg Natriumperborat-Monohydrat (PB*1) und 50 mg eines Aktivators wurde eine Bleichmittelzusammensetzung erhalten. Vier mit schwarzem Tee verschmutzte Gewebestücke (BC-1-Tee auf Baumwolle, 1,25 g, WFK) wurden für ein dreißigminütiges, isothermes Waschexperiment in einem Linitest-Gerät zugegeben. Die Gewebestücke wurden nach der vorgegebenen Waschzeit mit Wasser gespült, getrocknet und gebügelt. Anschließend wurde die Bleichwirkung mittels eines Weißgrad-Meßgerätes ELREPHO 2000 (Datacolor) festgestellt, indem die Unterschiede der Remissionen vor und nach dem Bleichen ermittelt wurden.

Vergleichsversuche, in denen der Waschmittelzusammensetzung zusätzlich 50 mg eines erfindungsgemäßen Aktivators zugegeben wurden, wurden durchgeführt. Die Untersuchungen wurden mit verschiedenen Anschmutzungen (z.B. Rotwein, Curry) wiederholt.

Es wurden Bleichmitteizusammensetzungen mit den Bleichaktivatoren 1 bis 3 hergestellt. Ihre Wirksamkeit wurde durch den Vergleich der Remissionen des Gewebes vor und nach dem Bleichvorgang ermittelt. Die Ergebnisse sind in Tabelle 1 angegeben. Die ΔΔ R-Werte geben die Verbesserung der Bleichwirkung der erfindungsgemäßen Zusammensetzung verglichen mit PB*1 an:

$$\Delta\Delta\ R\ (QUAT\text{-}PB^*1) = \Delta\ R\ (QUAT) - \Delta\ R\ (PB^*1)$$

Die Verbindungen 1 bis 3 sind

1 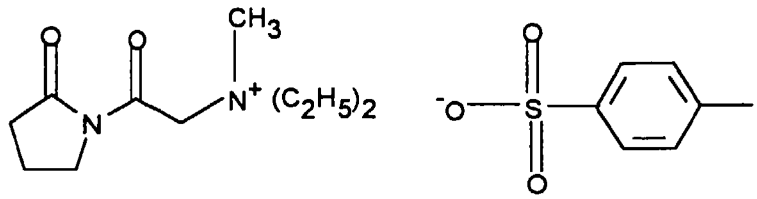

2 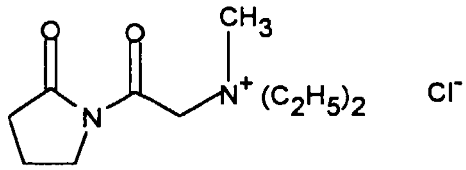

3 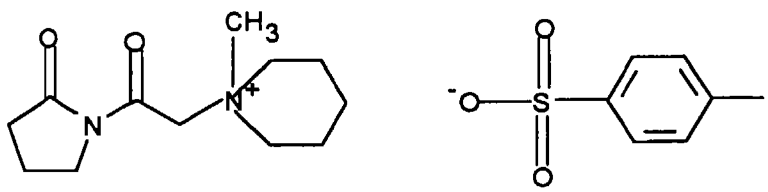

Tabelle 1:

| Aktivator Nr. | Δ R (QUAT) | ΔΔ R (QUAT-PB*1) |
|---|---|---|
| PB*1 | 7,8 | |
| 1 | 10,7 | 2,9 |
| 2 | 12,3 | 4,5 |
| 3 | 10,1 | 2,3 |

Tee, 40°C

| Aktivator Nr. | Δ R (QUAT) | ΔΔ R (QUAT-PB*1) |
|---|---|---|
| PB*1 | 15,1 | |
| 1 | 18,2 | 3,1 |
| 2 | 19,8 | 4,7 |
| 3 | 17,1 | 2,0 |

Rotwein, 40°C

Die Waschexperimente zeigen, daß die Bleichmittelzusammensetzungen, die erfindungsgemäße Bleichaktivatoren enthalten, bessere Waschergebnisse liefern als das Vergleichswaschmittel ohne erfindungsgemäße Bleichaktivatoren.

**Patentansprüche**

**1.** Verbindungen der Formel I

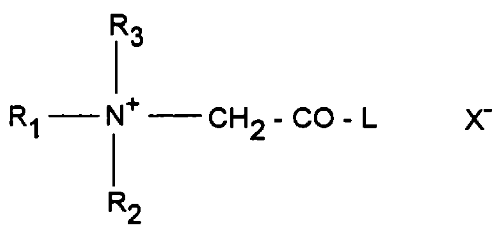

(I)

worin

a) $R_1$, $R_2$ und $R_3$ unabhängig voneinander $C_1$- bis $C_{24}$-Alkyl, Aryl, $C_2$-$C_{24}$-Alkenyl, $C_1$-$C_4$-Alkoxy-$C_1$-$C_4$-Alkyl, oder $CH_2$-CO-L sind,
oder
b) $R_2$ und $R_3$ zusammen mit dem Stickstoffatom, an das sie gebunden sind, einen Ring mit 4 bis 6 Kohlenstoffatomen bilden, wobei dieser Ring zusätzlich zum Stickstoffatom anstelle von Kohlenstoffatomen ein oder zwei Sauerstoffatome oder eine Gruppe

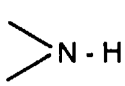

oder

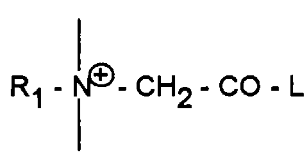

enthalten kann,

L eine Gruppe der Formeln

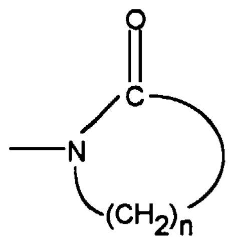

, oder

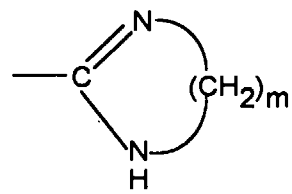

n eine Zahl von 3 bis 5, m eine Zahl von 2 bis 4 und X ein Anion ist.

2. Verbindungen nach Anspruch 1, dadurch gekennzeichnet, daß $R_1$, $R_2$ und $R_3$ unabhängig voneinander unsubstituiertes $C_2$- bis $C_4$-Alkenyl, unsubstituiertes $C_1$-$C_4$-Alkyl oder Phenyl sind.

3. Verbindungen nach einem oder mehreren der Ansprüche 1 oder 2, dadurch gekennzeichnet, daß L eine Pyrrolidongruppe ist.

4. Verbindungen nach einem oder mehreren der Ansprüche 1 bis 3, dadurch gekennzeichnet, daß X Chlorid, Bromid, Iodid, Fluorid, Sulfat, Hydrogensulfat, Carbonat, Hydrogencarbonat, Phosphat, Mono- und Di-Hydrogenphosphat, Pyrophosphat, Metaphosphat, Nitrat, Methosulfat, Dodecylsulfat, Dodecylbenzolsulfonat, Phosphonat, Methylphosphonat, Methandisulfonat, Methylsulfonat, Ethansulfonat oder p-Toluolsulfonat ist.

5. Verbindungen nach einem oder mehreren der Ansprüche 1 bis 4, dadurch gekennzeichnet, daß es sich um Trialkylammoniumacetylpyrrolidonchloride und Trialkylammoniumacetylimidazolchloride handelt.

6. Verbindungen nach einem oder mehreren der Ansprüche 1 bis 5, dadurch gekennzeichnet, daß es sich um (N,N,N-Trimethylammonium)-acetylpyrrolidonchlorid, 2-(N,N,N-Triethylammonium)-acetylpyrrolidonchlorid, 2-(N,N-Diethyl,N-methylammonium)-acetylpyrrolidonchlorid, 2-(N,N,N-Trimethylammonium)-acetylimidazolchlorid, 2-(N,N,N-Trimethyl-ammonium)-acetylimidazolchlorid oder 2-(N,N-Diethyl,N-methylammonium)-acetylimidazolchlorid handelt.

7. Wasch- und Reinigungsmittel, enthaltend

a) 2 bis 40 Gew.-% einer Peroxyverbindung
b) 0,1 bis 20 Gew.-% einer Verbindung nach einem oder mehreren der Ansprüche 1 bis 6 als Bleichaktivator.

8. Wasch- und Reinigungsmittel nach Anspruch 7, dadurch gekennzeichnet, daß die Peroxyverbindung in einer Menge von 4 bis 30 Gew.-% vorhanden ist.

9. Wasch- und Reinigungsmittel nach einem oder mehreren der Ansprüche 7 oder 8, dadurch gekennzeichnet, daß die Peroxyverbindung in einer Menge von 10 bis 25 Gew.-% vorhanden ist.

10. Wasch- und Reinigungsmittel nach einem oder mehreren der Ansprüche 7 bis 9, dadurch gekennzeichnet, daß der Bleichaktivator in einer Menge von 0.5 bis 10 Gew.-% vorhanden ist.

11. Wasch- und Reinigungsmittel nach einem oder mehreren der Ansprüche 7 bis 10, dadurch gekennzeichnet, daß der Bleichaktivator in einer Menge von 1 bis 7.5 Gew.-% vorhanden ist.

12. Wasch- und Reinigungsmittel nach einem oder mehreren der Ansprüche 7 bis 11, dadurch gekennzeichnet, daß die Perverbindung ein anorganisches Material ausgewählt aus der Gruppe bestehend aus Perborat, Percarbonat, Perphosphat, Persilikat und Monopersulfat ist.

13. Wasch- und Reinigungsmittel nach einem oder mehreren der Ansprüche 7 bis 12, dadurch gekennzeichnet, daß

die Perverbindung ein organisches Material ausgewählt aus der Gruppe bestehend aus Harnstoffperoxid, Cumolhydroperoxid und t-Butylhydroperoxid ist.

14. Wasch- und Reinigungsmittel nach einem oder mehreren der Ansprüche 7 bis 13, dadurch gekennzeichnet, daß sie 1 bis 80% eines Detergens-Gerüststoffes enthalten.

15. Wasch- und Reinigungsmittel nach einem oder mehreren der Ansprüche 7 bis 14, dadurch gekennzeichnet, daß sie eine zur Reinigung wirksame Menge eines Enzyms ausgewählt aus der Gruppe bestehend aus Proteasen, Cellulasen, Lipasen, Amylasen und Mischungen davon enthalten.

16. Wasch- und Reinigungsmittel nach einem oder mehreren der Ansprüche 7 bis 15, dadurch gekennzeichnet, daß sie oberflächenaktive Substanzen in einer Menge von bis zu 50 Gew.-% enthalten.

17. Wasch- und Reinigungsmittel nach einem oder mehreren der Ansprüche 7 bis 16, dadurch gekennzeichnet, daß sie Seifen in einer Menge von bis zu 25 Gew.-% enthalten.

18. Wasch- und Reinigungsmittel nach einem oder mehreren der Ansprüche 7 bis 17, dadurch gekennzeichnet, daß sie einen Builder in einer Menge von 5 bis 80 Gew.-% enthalten.

19. Verfahren zur Herstellung von Verbindungen nach einem oder mehreren der Ansprüche 1 bis 6, dadurch gekennzeichnet, daß man im ersten Schritt ein Lactam mit Chloracetylchlorid in Gegenwart einer Hilfsbase zu einem N-Chloracetylamid umsetzt, letzteres im zweiten Schritt mit einem sekundären Amin zu einem 2-(N,N-Dialkylamin)-Acetyllactam umsetzt, das im dritten Schritt zu einer Verbindung nach einem oder mehreren der Ansprüche 1 bis 6 alkyliert wird.

20. Verfahren zur Herstellung von Verbindungen nach einem oder mehreren der Ansprüche 1 bis 6, dadurch gekennzeichnet, daß man im ersten Schritt ein Lactam mit Chloracetylchlorid in Gegenwart einer Hilfsbase zu einem N-Chloracetylamid umsetzt, und letzteres im zweiten Schritt mit einem tertiären Amin zu einer Verbindung gemäß einem der Ansprüche 1 bis 6 umsetzt.

Europäisches Patentamt

# EUROPÄISCHER RECHERCHENBERICHT

Nummer der Anmeldung
EP 97 10 9809

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| X | JP 02 115 154 A (KAO CORP.)<br>* Seite 454 (Seite 6 des Patentdokuments), Formeln (I-1), (I-2) *<br>--- | 1-6 | C07D207/26<br>C07D233/22<br>C11D3/39 |
| A,D | EP 0 371 809 A (KAO CORP.)<br>* Ansprüche 1,5 *<br>--- | 1,7,14 | |
| A,D | EP 0 402 971 A (UNILEVER)<br>* Ansprüche 1,19 *<br>--- | 1,7,14, 16,18 | |
| A,D | US 4 751 015 A (R. W. HUMPHREYS ET AL.)<br>* Ansprüche 1,18 *<br>--- | 1,7 | |
| A,D | US 4 933 103 A (M. AOYAGI ET AL.)<br>* Ansprüche 1,8,10,12 *<br>--- | 1,7,13, 16 | |
| A,D | EP 0 403 152 A (KAO CORP.)<br>* Ansprüche 1,4 *<br>--- | 1,7,15, 16 | RECHERCHIERTE SACHGEBIETE (Int.Cl.6) |
| A,D | EP 0 427 224 A (KAO CORP.)<br>* Ansprüche 1,2 *<br>--- | 1,7 | C07D<br>C11D |
| A,D | US 5 460 747 A (E. P. GOSSELINK ET AL.)<br>* Ansprüche 1,6,10,11,15 *<br>----- | 1,7,14, 16,18 | |

Der vorliegende Recherchenbericht wurde für alle Patentansprüche erstellt

| Recherchenort | Abschlußdatum der Recherche | Prüfer |
|---|---|---|
| BERLIN | 10.Oktober 1997 | Hass, C |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)